# EUROPÄISCHE PATENTSCHRIFT

(11) **EP 3 651 703 B1**
(45) Veröffentlichungstag und Bekanntmachung des Hinweises auf die Patenterteilung: **23.03.2022**
(21) Anmeldenummer: 18734543.4
(22) Anmeldetag: 26.06.2018
(51) Int. Cl.: A61F 5/01

(54) **VERBINDUNGSEINRICHTUNG UND GELENKEINRICHTUNG FÜR EINE ORTHOPÄDIETECHNISCHE EINRICHTUNG**
CONNECTION DEVICE AND JOINT DEVICE FOR A TECHNICAL ORTHOPEDIC DEVICE
DISPOSITIF DE LIAISON ET DISPOSITIF D'ARTICULATION POUR UN DISPOSITIF ORTHOPÉDIQUE

(30) Priorität: 11.07.2017 DE 102017115560
(43) Veröffentlichungstag der Anmeldung: 20.05.2020
(73) Patentinhaber: Ottobock SE & Co. KGaA, 37115 Duderstadt (DE)
(72) Erfinder: SCHMITT, Alexander, 34123 Kassel (DE); HORSTMANN, Klaus, 48485 Neuenkirchen (DE)
(74) Vertreter: Gramm, Lins & Partner Patent- und Rechtsanwälte PartGmbB
(86) Internationale Anmeldenummer: PCT/EP2018/067165
(87) Internationale Veröffentlichungsnummer: WO 2019/011651

(56) Entgegenhaltungen:
- WO-A1-01/21114
- WO-A1-2016/131571
- DE-T2- 60 035 431
- US-A1- 2006 206 045

## Beschreibung

Die Erfindung betrifft eine Verbindungseinrichtung zum Verbinden eines ersten Bauteiles mit einem zweiten Bauteil einer orthopädietechnischen Einrichtung, wie in den Ansprüchen definiert.

Orthopädietechnische Einrichtungen können beispielsweise Orthesen oder Prothesen sein. Diese Einrichtungen bestehen aus einer Vielzahl unterschiedlicher Bauelemente, beispielsweise Schienenelemente, die miteinander verbunden werden müssen. Dazu sind aus dem Stand der Technik eine Reihe unterschiedlicher Verbindungseinrichtungen bekannt.

Insbesondere durch Orthesen sollen oftmals Kräfte auf bestimmte Körperteile des Trägers der Orthese ausgeübt werden. So kann beispielsweise bei einer Knieorthese, die zur Linderung von Osteoarthritis-Beschwerden eingesetzt wird, eine in medialer Richtung rückende Kraft auf das Knie des Trägers ausgeübt werden. Damit das Knie während des Tragens der Orthese gebeugt werden kann, müssen dabei zwei Bauteile, nämlich eine Oberschenkelschiene und eine Unterschenkelschiene, im Bereich des Knies so miteinander verbunden werden, dass eine Beugung des Knies ermöglicht wird, gleichzeitig jedoch eine Kraft in medialer Richtung auf das Knie aufgebracht werden kann. Um diese Kraft aufzubringen sind aus dem Stand der Technik unterschiedlichste Ausführungsformen einer entsprechenden Verbindungseinrichtung bekannt. So werden beispielsweise aufblasbare oder mit einem Fluid füllbare Kissen verwendet, die zwischen der eigentlichen Verbindungseinrichtung und dem Knie angeordnet werden. Alternativ dazu sind aus der DE 600 35431 T2 Gelenke für orthopädische Knieorthesen bekannt, bei denen eine Oberschenkelschale und eine Unterschenkelschale vorhanden sind, deren Winkel zum restlichen Schienensystem einstellbar ist. Aus auf diese Weise lässt sich die Kraft auf das Knie einstellen.

Weitere Möglichkeiten, den Winkel einzustellen, sind aus der WO 01/21114 A1 und der US 2006/0206045 A1 bekannt.

Nachteilig ist jedoch, dass die Einstellmöglichkeit trotz eines relativ großen Winkelversatzes zwischen den einzelnen Bauteilen relativ gering ist und der zur Verfügung stehende Hebelarm relativ klein ist.

Die Erfindung liegt daher die Aufgabe zugrunde, eine möglichst genaue und über einen großen Kraftbereich verstellbare Einstellbarkeit eines Winkels zwischen zwei miteinander verbindbaren Bauteilen bereitzustellen, die den Nachteil aus dem Stand der Technik überwindet oder zumindest abmildert.

Die Erfindung löst die gestellte Aufgabe durch eine Verbindungseinrichtung zum Verbinden eines ersten Bauteils mit einem zweiten Bauteil einer orthopädietechnischen Einrichtung, wobei die Verbindungseinrichtung ein erstes Schwenkelement zum Anordnen an dem ersten Bauteil und ein zweites Schwenkelement zum Anordnen an dem zweiten Bauteil aufweist, wobei an dem ersten Schwenkelement ein erstes Einstellelement angeordnet ist, dass eine Kontaktfläche aufweist, und wobei das zweite Schwenkelement eine zweite Kontaktfläche aufweist, wobei das erste Schwenkelement an dem zweiten Schwenkelement um eine Schwenkachse schwenkbar angeordnet ist und das erste Einstellelement derart angeordnet und ausgebildet ist, dass die erste Kontaktfläche an der zweiten Kontaktfläche anliegt, wobei das erste Schwenkelement um eine Drehachse drehbar angeordnet ist, wobei ein Abstand der ersten Kontaktfläche von der Drehachse variiert.

Bei einer erfindungsgemäßen Verbindungseinrichtung sind folglich die beiden Schwenkelemente schwenkbar aneinander angeordnet. Durch eine Verschwenkung um diese Schwenkachse kann der Winkel zwischen den beiden miteinander zu verbindenden Bauteilen eingestellt und verändert werden.

Die erste Kontaktfläche, die an dem ersten Einstellelement am ersten Schwenkelement angeordnet ist, liegt bei einer erfindungsgemäßen Verbindungseinrichtung an der zweiten Kontaktfläche, die an dem zweiten Schwenkelement angeordnet ist an. Die Position der ersten Kontaktfläche ist dabei entscheidend für den Winkel, der zwischen den beiden Schwenkelementen eingeschlossen ist, wenn sie aneinander anliegen.

In einer bevorzugten Ausgestaltung ist das erste Einstellelement lösbar an dem ersten Schwenkelement angeordnet. Dadurch lässt es sich leicht entfernen und beispielsweise gegen ein anderes erstes Einstellelement austauschen, dass eine etwas anders geformte oder eine etwas anders positionierte erste Kontaktfläche aufweist, sodass ein Winkel zwischen dem ersten Schwenkelement und dem zweiten Schwenkelement durch den Austausch der ersten Einstellelemente gegeneinander angepasst und verändert werden kann. Das Schwenkelement kann eingeklippst, eingesteckt, eingeschraubt oder auf sonstige Weise formschlüssig oder auf andere Weise lösbar am ersten Schwenkelement angeordnet sein.

Erfindungsgemäß ist das erste Einstellelement um eine Drehachse drehbar angeordnet, wobei ein radialer Abstand der ersten Kontaktfläche von der Drehachse variiert.

Wird nun das erste Einstellelement um die Drehachse gedreht, verändert sich der Bereich der ersten Kontaktfläche, der mit der zweiten Kontaktfläche in Kontakt kommt. Dieser Bereich verschiebt sich entlang der ersten Kontaktfläche. Da erfindungsgemäß der radiale Abstand der ersten Kontaktfläche von der Drehachse variiert hat eine Drehung des ersten Einstellelementes eine Verschwenkung des ersten Schwenkelementes relativ zum zweiten Schwenkelement um die Schwenkachse zur Folge, wodurch ein Winkel zwischen den beiden zu verbindenden Bauteilen verändert wird. Durch eine Drehung des ersten Einstellelementes lässt sich folglich einfach, reproduzierbar und sicher der Winkel zwischen den beiden zu verbindenden Bauteilen einstellen.

Bevorzugt verfügt das erste Einstellelement über eine Werkzeugschnittstelle, beispielsweise einem Innensechskant oder einen Innenvierkant, die eingerichtet ist, mit einem entsprechenden Werkzeug zusammenzuwirken. Wenn zum Drehen des ersten Einstellelementes ein Werkzeug benötigt wird, wird ein versehentliches Bewegen und damit ein versehentliches Verstellen verhindert. Durch das versehentliche Verstellen könnte ein Winkel zwischen den zu verbindenden Bauteilen und damit gegebenenfalls eine auf einen Körperteil aufbringbare Kraft versehentlich verstellt und gegebenenfalls aus dem therapeutisch sinnvollen Bereich herausbewegt werden. Dies wird auf diese Weise sicher verhindert.

Vorteilhafterweise variiert der radiale Abstand der ersten Kontaktfläche von der Drehachse in Umfangsrichtung zumindest über einen Teil des Umfangs. Vorteilhafterweise variiert er über den gesamten Umfang. Auf diese Weise kann sichergestellt werden, dass eine Rotation des ersten Einstellelementes um die Drehachse immer eine Veränderung des Abstandes zwischen der zweiten Kontaktfläche, die an der ersten Kontaktfläche anliegt, und der Drehachse zur Folge hat, sodass es zu einem Verschwenken der beiden Schwenkelemente um die Schwenkachse kommt. Dies gilt solange sich der radiale Abstand der ersten Kontaktfläche von der Drehachse in dem Bereich der ersten Kontaktfläche, der an der zweiten Kontaktfläche anliegt, bei einer Rotation des ersten Einstellelementes ändert.

Vorzugsweise ist die erste Kontaktfläche zumindest in einem Bereich relativ zu der Drehachse geneigt. Vorteilhafterweise ist sie überall relativ zur Drehachse geneigt. Dies umfasst auch den Fall, bei dem die Neigung das Vorzeichen, also die Richtung wechselt, so dass an einer oder an wenigen Stellen die erste Kontaktfläche parallel zur Drehachse verläuft. Auf diese Weise ist es möglich, einen relativ großen Bereich der ersten Kontaktfläche tatsächlich in Kontakt mit der zweiten Kontaktfläche zu bringen. Durch geschickte Wahl der Neigung ist es möglich, dass sich die Größe dieses Kontaktbereiches der beiden Kontaktflächen nicht ändert. Je größer dieser Kontaktbereich der beiden Kontaktflächen ist, in dem die beiden Kontaktflächen miteinander in Kontakt stehen, desto mehr Kraft kann bei einer Rotation des Einstellelementes übertragen werden, sodass eine Verstellung der Schwenkposition der beiden Schwenkelemente relativ zueinander besonders einfach und sicher möglich wird.

In einer bevorzugten Ausgestaltung der Verbindungseinrichtung ist das erste Bauteil und/oder das zweite Bauteil relativ zu der Verbindungseinrichtung schwenkbar, wenn es an dem jeweiligen Schwenkelement angeordnet ist. Auf diese Weise kann das Verbindungselement gleichzeitig die Funktion eines Gelenkes übernehmen. Dies ist insbesondere bei Knieorthesen, jedoch auch bei anderen orthopädietechnischen Einrichtungen von Vorteil. Die auf das Körperteil aufzubringende Kraft hängt unter anderem vom Hebelarm, also insbesondere der Länge der beiden miteinander zu verbindenden Bauteile bis zur nächsten Kontaktstelle mit dem Körper des Trägers der Orthese oder Prothese ab. Wird nun eine Verbindungseinrichtung gemäß einem Ausführungsbeispiel der vorliegenden Erfindung, die gleichzeitig die Funktion eines Gelenkes übernehmen kann, verwendet, kann diese beispielsweise bei einer Knieorthese direkt neben oder zumindest nahe des Knies und insbesondere der Knieachse angeordnet werden. Die beiden miteinander zu verbindenden Bauteile können in diesem Fall beispielsweise Schienenelemente sein, die zu einem Oberschenkel- und einem Unterschenkelgurt oder einer entsprechenden Schale führen. Damit wird die gesamte Länge der beiden Bauteile zum Hebel, über den die Kraft beispielsweise bei einer Dreipunkt-Wirkung übertragen werden kann. Selbst bei einer relativ geringen Winkeländerung hat dies eine relativ große Änderung der aufgebrachten Kräfte zur Folge, sodass die aufzubringende Kraft über einem relativ großen Bereich einstellbar ist.

Besonders bevorzugt ist die Ausgestaltung, in der beide Bauteile relativ zur Verbindungseinrichtung verschwenkbar sind.

In einer bevorzugten Ausgestaltung ist das erste Einstellelement drehfest mit dem ersten Bauteil verbindbar. In dieser besonderen Ausgestaltung wird folglich das erste Schwenkelement relativ zum zweiten Schwenkelement verschwenkt, wenn das erste Bauteil relativ zur Verbindungseinrichtung und insbesondere relativ zum zweiten Bauteil verschwenkt wird. Bei einer Knieorthese findet dies beispielsweise beim Gehen statt, wenn das Knie gebeugt wird. Dies hat zur Folge, dass in diesem Beispiel die auf das Knie aufgebrachte Kraft im Laufe eines Schrittzyklus variiert und dieser Kraftverlauf durch die geeignete Wahl der Form und Anordnung der ersten Kontaktfläche individuell eingestellt werden kann. Die aufgebrachte Kraft kann im Laufe des Schrittes zunehmen, abnehmen oder bereichsweise konstant bleiben. Dies kann eingestellt werden, in dem ein Verlauf des radialen Abstands der ersten Kontaktfläche von der Drehachse eingestellt wird. Bleibt der radiale Abstand über einen Umfangsbereich um die Drehachse konstant, führt auch eine Drehung des Einstellelementes nicht zu einer Verschwenkung der beiden Schwenkelemente gegeneinander und somit auch nicht zu einer Änderung der aufzubringenden Kraft. Ändert sich jedoch der radiale Abstand der ersten Kontaktfläche von der Drehachse des Einstellelementes im Schwenkbereich, der durch die Bewegung des Körperteils abgedeckt wird, führt diese Änderung des Abstandes zu einem Verschwenken der beiden Schwenkelemente relativ zueinander und damit auch zu einer Veränderung der aufgebrachten Kraft.

Vorteilhafterweise ist an dem zweiten Schwenkelement ein zweites Einstellelement angeordnet, das die zweite Kontaktfläche aufweist und vorzugsweise lösbar und/oder um eine zweite Drehachse drehbar angeordnet ist. Die beiden Kontaktflächen sind in diesem Ausführungsbeispiel folglich jeweils an einem drehbaren Einstellelement angeordnet, wodurch ein Einstellbereich der Schwenkposition der beiden Schwenkelemente und damit der auf ein Körperteil aufzubringenden Kraft weiter vergrößert wird.

Das erste Einstellelement und/oder das zweite Einstellelement sind vorteilhafterweise mehrteilig ausgebildet. So ist es denkbar, ein Basisteil zu verwenden, das um umfangseitig mit Formschlusselementen, beispielsweise hervorstehenden Zacken oder Stift, ausgebildet ist. Diese können mit einem Ringelement verbunden werden, das über entsprechende Formschlusselemente verfügt. Das Ringelement kann beispielsweise leicht austauschbar oder entfernbar sein. Auf diese Weise kann ein bestehendes Verbindungselement leicht auf individuelle Anforderungen und beispielsweise therapeutische Notwendigkeiten angepasst werden, ohne dass ein vollständig neues Verbindungselement verwendet werden muss oder ein Einstellelement aufwendig aus- und umgebaut werden muss. Es wird lediglich das Ringelement entfernt und durch ein anderes passendes Ringelement ersetzt oder an die gewünschten Anforderungen angepasst.

Die Erfindung löst die gestellte Aufgabe zudem durch eine Gelenkeinrichtung für eine orthopädietechnische Einrichtung, wobei die Gelenkeinrichtung ein erstes Bauteil, ein zweites Bauteil und eine Verbindungseinrichtung der hier beschriebenen Art aufweist, wobei das erste Bauteil an dem ersten Schwenkelement und das zweite Bauteil an dem zweiten Schwenkelement derart angeordnet sind, dass das erste Bauteil relativ zu dem zweiten Bauteil um eine Gelenkachse verschwenkbar ist, wobei die Gelenkachse vorzugsweise einen rechten Winkel mit der Schwenkachse einschließt. Derartige Gelenkeinrichtungen sind insbesondere für Knieorthesen, jedoch auch für andere orthopädietechnische Einrichtungen von Vorteil.

Vorzugsweise sind dabei das erste Bauteil und das zweite Bauteil derart miteinander verbunden, dass durch ein Verschwenken eines der beiden Bauteile relativ zu der Verbindungseinrichtung das jeweils andere der beiden Bauteile relativ zu der Verbindungseinrichtung verschwenkt wird. Dies kann vorteilhafterweise dadurch erreicht werden, dass die beiden Bauteile jeweils eine Mehrzahl von Zähnen aufweisen, die ineinander eingreifen und kämmen. Alternativ oder zusätzlich dazu können die beiden Bauteile durch wenigstens ein, bevorzugt wenigstens zwei Zugkraftübertragungselemente miteinander verbunden sein.

Vorteilhafterweise ist ein maximaler Verschwenkwinkel des ersten Bauteils relativ zu dem zweiten Bauteil in wenigstens einer Schwenkrichtung durch einen einstellbaren Endanschlag begrenzt, der vorzugsweise einstellbar ist. Besonders vorteilhafter ist ein maximaler Schwenkwinkel in beiden Schwenkrichtungen durch jeweils eine in vorzugsweise einstellbaren Endanschlag begrenzt. Auf diese Weise kann das Gelenk individuell angepasst und an die jeweiligen Bedürfnisse eingestellt werden.

Mit Hilfe der beiliegenden Zeichnungen wird nachfolgend ein Ausführungsbeispiel der vorliegenden Erfindung näher erläutert. Es zeigt
- Figur 1-: die schematische Darstellung einer Gelenkeinrichtung gemäß einem ersten Ausführungsbeispiel der vorliegenden Erfindung in zwei Positionen,
- Figur 2-: eine Explosionsdarstellung eines Teils einer Gelenkeinrichtung,
- Figur 3-: drei schematische Schnittdarstellungen durch eine Gelenkeinrichtung in unterschiedlichen Positionen.
- Figur 4-: eine schematische Teilschnittdarstellung durch eine Gelenkeinrichtung,
- Figur 5-: eine schematische Explosionsdarstellung durch eine Gelenkeinrichtung gemäß einem weiteren Ausführungsbeispiel der vorliegenden Erfindung,
- Figur 6-: eine Draufsicht und eine perspektivische Ansicht einer Gelenkeinrichtung gemäß einem weiteren Ausführungsbeispiel der vorliegenden Erfindung,
- Figur 7-: zwei Darstellungen jeweils einer Orthese, die über eine Gelenkeinrichtung gemäß einem Ausführungsbeispiel der vorliegenden Erfindung verfügt,
- Figur 8-: die schematische Darstellung einer weiteren Orthese mit einer Gelenkeinrichtung und
- Figur 9-: die schematische Darstellung einer weiteren Gelenkeinrichtung gemäß einem weiteren Ausführungsbeispiel der vorliegenden Erfindung.

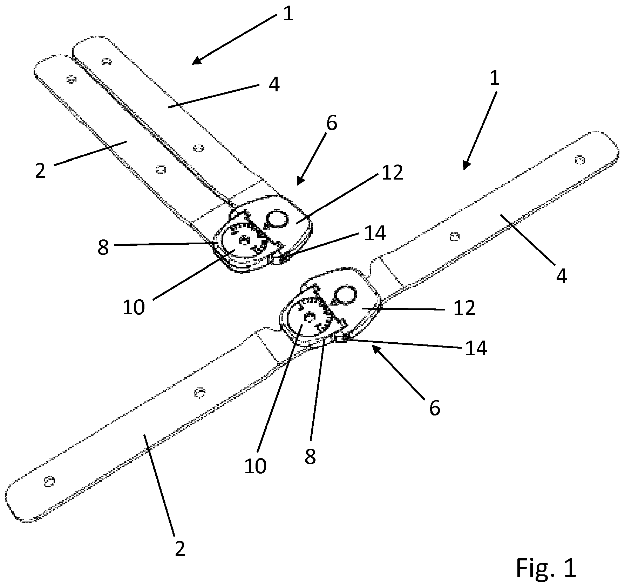

Figur 1 zeigt eine Gelenkeinrichtung 1 gemäß einem ersten Ausführungsbeispiel der vorliegenden Erfindung in zwei unterschiedlichen Positionen. Sie verfügt über ein erstes Bauteil 2 sowie ein zweites Bauteil 4. Beide sind an einer Verbindungseinrichtung 6 angeordnet, die über ein erstes Schwenkelement 8 mit einem ersten Einstellelement 10 sowie ein zweites Schwenkelement 12 verfügt. Das erste Schwenkelement 8 und das zweite Schwenkelement 12 sind um eine Schwenkachse gegeneinander verschwenkbar.

Man erkennt in den beiden Darstellungen aus Figur 1, dass das erste Bauteil 2 und das zweite Bauteil 4 relativ zueinander um eine Gelenkachse, die nicht dargestellt ist, verschwenkbar sind. Dies hat im vorliegenden Ausführungsbeispiel kein Verschwenken des ersten Schwenkelementes 8 relativ zum zweiten Schwenkelement 12 um die Schwenkachse 14 zur Folge.

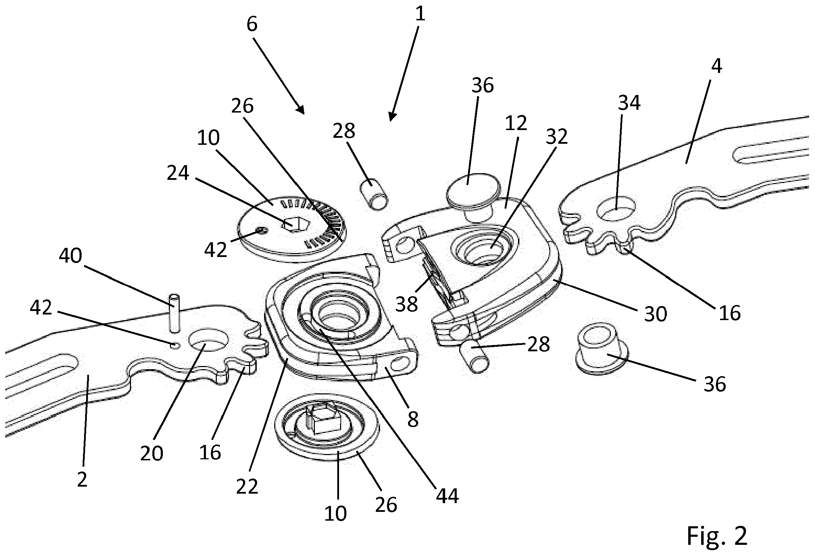

Figur 2 zeigt eine Explosionsdarstellung durch eine Gelenkeinrichtung 1. Man erkennt das erste Bauteil 2 und das zweite Bauteil 4, die jeweils über mehrere Zähne 16 verfügen, die miteinander in Eingriff stehen, wenn die beiden Bauteile 2, 4 an der Verbindungseinrichtung 6 angeordnet sind. Das erste Schwenkelement 8 verfügt über eine zentrale Bohrung 18, die im montierten Zustand in Überdeckung mit einem Loch 20 gebracht wird, das im ersten Bauteil 2 angeordnet ist. Dazu wird das erste Bauteil 2 in einen Schlitz 22 eingeführt, der im ersten Schwenkelement 8 vorhanden ist. Das erste Einstellelement 10 besteht im vorliegenden Ausführungsbeispiel aus zwei tellerförmigen Elementen, die durch die zentrale Bohrung 18 und das Loch 20 hindurch miteinander verbunden werden können. Im oberen ist eine Ausnehmung 24 enthalten, die mit einem Werkzeug in Eingriff gebracht werden kann, um das erste Einstellelement 10 um eine Drehachse, die durch die zentrale Bohrung 18 hindurchverläuft, zu drehen.

Das erste Einstellelement 10 verfügt über die erste Kontaktfläche 26, deren radialer Abstand von einer Drehachse des ersten Einstellelementes 10 über den Umfang des ersten Einstellelementes 10 hinweg variiert.

Zwei Stifte 28, die durch entsprechende Ausnehmungen hindurchgesteckt werden, verbinden die beiden Schwenkelemente 8, 12 schwenkbar miteinander. Durch ihre Mitte verläuft die Schwenkachse.

Auch das zweite Schwenkelement 12 verfügt über einen Schlitz 30, in den das zweite Bauteil 4 eingeführt wird, um eine zentrale Bohrung 32 mit einem Loch 34 in Überdeckung zu bringen, das im zweiten Bauteil 4 angeordnet ist. Anschließend werden Verbindungsstücke 36 hindurchgesteckt und das zweite Bauteil 4 so am zweiten Schwenkelement 12 angeordnet. Das zweite Schwenkelement verfügt über eine zweite Kontaktfläche 38.

Anders als im in Figur 1 gezeigten Ausführungsbeispiel verfügt die Gelenkeinrichtung 1 gemäß Figur 2 über einen Verbindungsstift 40 der über zwei Löcher 42 sowohl mit dem ersten Bauteil 2 als auch mit dem ersten Einstellelement 10 drehfest verbunden werden kann. Er kann in einem Langloch 44 im ersten Schwenkelement 8 entlanggleiten. Wird nun das erste Bauteil 2 relativ zum ersten Schwenkelement 8 bewegt, bewegt sich gleichzeitig das erste Einstellelement 10, sodass es zu einer Verschiebung der ersten Kontaktfläche 26 relativ zur zweiten Kontaktfläche 38 und damit zu einem Verschwenken um die Schwenkachse durch die beiden Stifte 28 kommt.

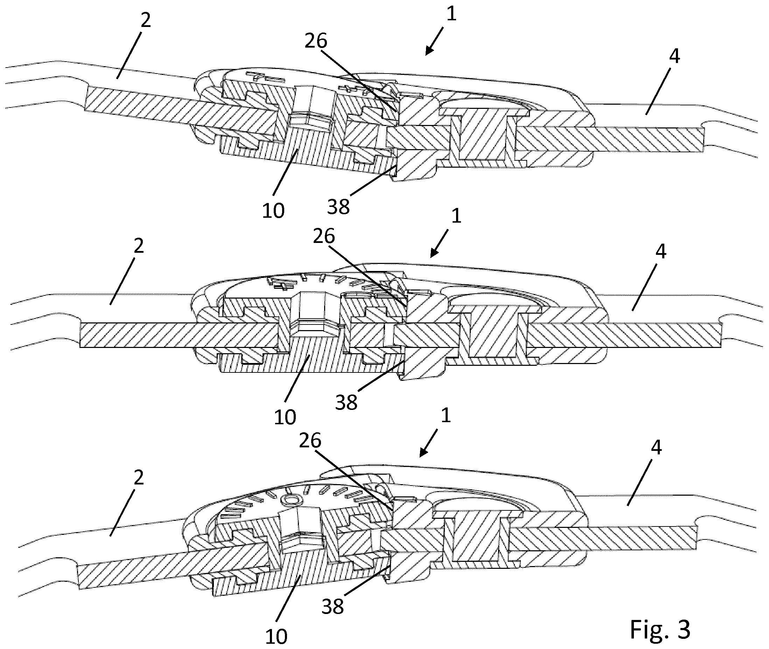

Dies ist in Figur 3 dargestellt. Man erkennt drei Schnittdarstellungen durch die Gelenkeinrichtung ein wobei jeweils zwischen dem ersten Bauteil 2 und dem zweiten Bauteil 4 unterschiedliche Schwenkwinkel um die Schwenkachse vorliegen. Dies liegt an den unterschiedlichen radialen Abständen und Neigungen der ersten Kontaktfläche 26 relativ zur zweiten Kontaktfläche 38. Wird das erste Einstellelement 10 um die Drehachse herum gedreht, verändert sich die Neigung und der radiale Abstand der ersten Kontaktfläche 26 in dem Bereich, der mit der zweiten Kontaktfläche 38 in Eingriff steht, und es kommt zur gezeigten Verschwenkung.

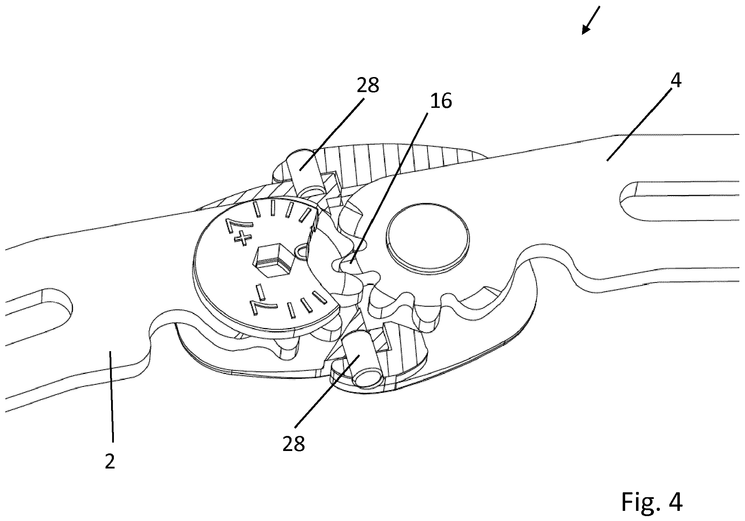

Figur 4 zeigt eine Teilschnittdarstellung. Man erkennt die beiden Stifte 28, die die Schwenkachse bilden sowie die Zähne 16 der beiden Bauteile 2, 4, die in Eingriff miteinander stehen.

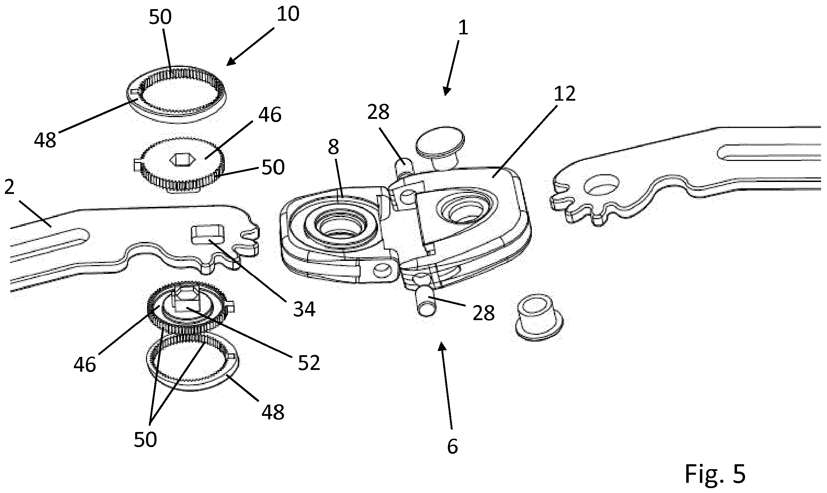

Figur 5 zeigt eine weitere Explosionsdarstellung durch eine Gelenkeinrichtung 1 gemäß einem weiteren Ausführungsbeispiel der vorliegenden Erfindung. Der Unterschied zu der in Figur 1 gezeigten Darstellung besteht einerseits darin, dass die in Figur 5 gezeigte Ausführungsform keinen Verbindungsstift 40 aufweist. Zudem ist das erste Einstellelement 10 anders aufgebaut. Die beiden Teile, die das erste Einstellelement 10 bilden, verfügen über jeweils einen Zentralanteil 46, an dem jeweils ein Ringelement 48 angeordnet ist. Dieses ist leicht austauschbar und über Formschlusselemente 50 am jeweiligen Zentralteil 46 befestigbar.

Auch bei der in Figur 5 gezeigten Ausgestaltung ist durch die Ausgestaltung des Loches 34 und des entsprechend ausgestalteten Verbindungsendes 52 des ersten Einstellelementes 10 eine formschlüssige Verbindung zwischen dem ersten Einstellelement 10 und dem ersten Bauteil 2 hergestellt, sodass auch hier eine Verschwenkung des ersten Bauteils 2 relativ zur Verbindungseinrichtung 6 eine Veränderung des Neigungswinkels und eine Verschwenkung des ersten Schwenkelementes 8 relativ zum zweiten Schwenkelement 12 um die Schwenkachse, die durch die beiden Stifte 28 verläuft, zur Folge hat.

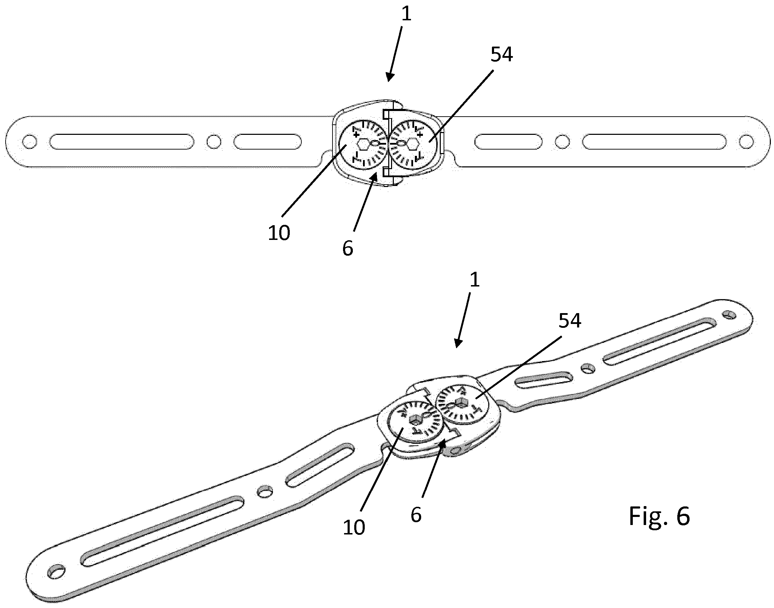

Figur 6 zeigt zwei Darstellungen einer weiteren Ausführungsform einer Gelenkeinrichtung, bei der die Verbindungseinrichtung 6 über das erste Einstellelement 10 sowie ein zweites Einstellelement 54 verfügt. Eine äußere Mantelfläche des zweiten Einstellelementes 54 bildet die zweite Kontaktfläche 38.

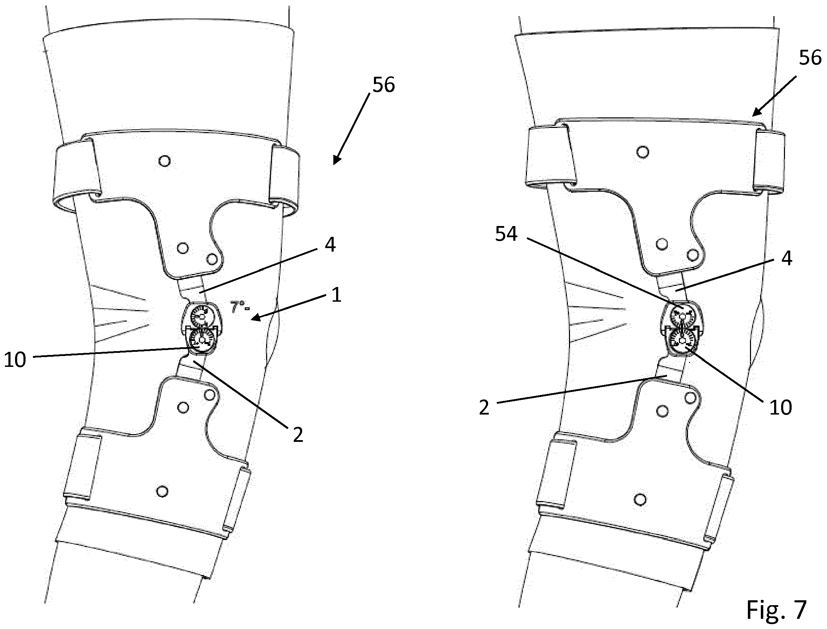

Figur 7 zeigt zwei Orthesen 56, die als Knieorthesen ausgebildet sind und jeweils eine Gelenkeinrichtung 1 aufweisen. Sie ist zwischen den beiden Bauteilen 2, 4 angeordnet. Die Schwenkachse verläuft in der Zeichenebene. Man erkennt an der Stellung der beiden Einstellelemente 10, 54 unterschiedliche Einstellungen, sodass ein Winkel zwischen den beiden Bauteilen 2, 4 unterschiedlich ausgebildet ist.

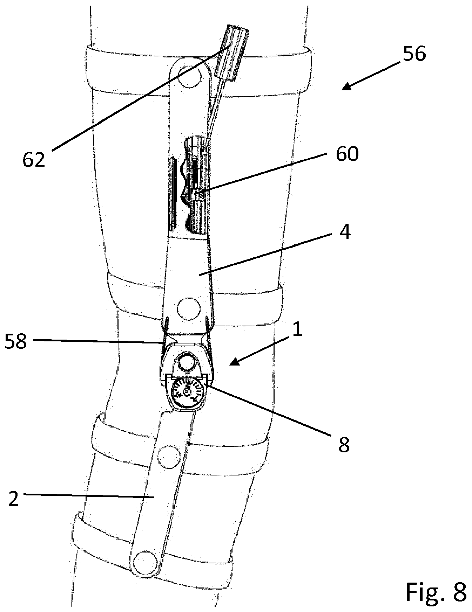

Figur 8 zeigt eine weitere Orthese 56, die über eine Gelenkeinrichtung 1 verfügt.

Zusätzlich verfügt sie über ein Zugelement 58 dessen Endanschläge 60 mit dem gezeigten Werkzeug 62 verstellbar sind. Ein Verschwenken der beiden Bauteile 2, 4 relativ zueinander hat eine Bewegung des Zugelementes 58, das mit beiden Enden am zweiten Bauteil 4 angeordnet ist und im gezeigten Ausführungsbeispiel fest mit dem ersten Schwenkelement 8 verbunden ist, zur Folge. Dadurch verschieben sich die Enden, die nicht dargestellt sind, des Zugelementes 58 bis sie gegebenenfalls an einem der Endanschläge 60 anschlagen.

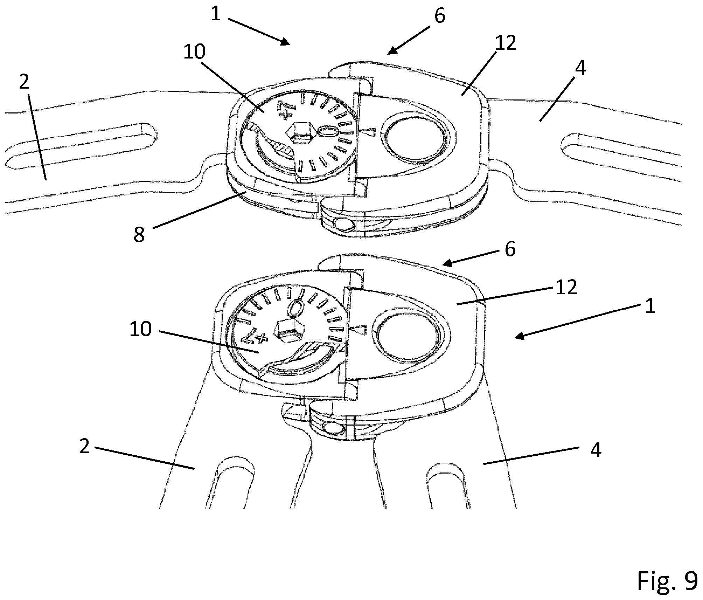

Figur 9 zeigt zwei Teilschnittdarstellungen einer Gelenkeinrichtung 1. Man erkennt an den beiden Darstellungen, dass ein Verschwenken der beiden Bauteile 2, 4 relativ zueinander und relativ zur Verbindungseinrichtung 6 eine Verschwenkung der beiden Schwenkelemente 8, 12 relativ zueinander um die Schwenkachse zur Folge hat.

### Bezugszeichenliste:

- 1: Gelenkeinrichtung
- 2: erstes Bauteil
- 4: zweites Bauteil
- 6: Verbindungseinrichtung
- 8: erstes Schwenkelement
- 10: erstes Einstellelement
- 12: zweites Schwenkelement
- 14: Schwenkachse
- 16: Zahn
- 18: zentrale Bohrung
- 20: Loch
- 22: Schlitz
- 24: Ausnehmung
- 26: erste Kontaktfläche
- 28: Stift
- 30: Schlitz
- 32: zentrale Bohrung
- 34: Loch
- 36: Verbindungsstück
- 38: zweite Kontaktfläche
- 40: Verbindungsstift
- 42: Loch
- 44: Langloch
- 46: Zentralanteil
- 48: Ringelement
- 50: Formschlusselement
- 52: Verbindungsende
- 54: zweites Einstellelement
- 56: Orthese
- 58: Zugelement
- 60: Endanschlag
- 62: Werkzeug

## Patentansprüche

1. Verbindungseinrichtung (6) zum Verbinden eines ersten Bauteiles (2) mit einem zweiten Bauteil (4) einer orthopädietechnischen Einrichtung, wobei die Verbindungseinrichtung (6)
- ein erstes Schwenkelement (8) zum Anordnen an dem ersten Bauteil (2) und
- ein zweites Schwenkelement (12) zum Anordnen an dem zweiten Bauteil (4) aufweist,
wobei
- an dem ersten Schwenkelement (8) ein erstes Einstellelement (10) angeordnet ist, das eine erste Kontaktfläche (26) aufweist,
- das zweite Schwenkelement (12) eine zweite Kontaktfläche (38) aufweist
wobei das erste Schwenkelement (8) an dem zweiten Schwenkelement (12) um eine Schwenkachse (14) schwenkbar angeordnet ist und das erste Einstellelement (10) derart angeordnet und ausgebildet ist, dass die erste Kontaktfläche (26) an der zweiten Kontaktfläche (38) anliegt, dadurch charakterisiert, dass das erste Einstellelement (10) um eine Drehachse drehbar angeordnet ist, wobei ein radialer Abstand der ersten Kontaktfläche (26) von der Drehachse variiert.

2. Verbindungseinrichtung (6) nach Anspruch 1, **dadurch gekennzeichnet, dass** das erste Einstellelement (10) lösbar an dem ersten Schwenkelement (8) angeordnet ist.

3. Verbindungseinrichtung (6) nach einem der vorstehenden Ansprüche, **dadurch gekennzeichnet, dass** der radiale Abstand der ersten Kontaktfläche (26) von der Drehachse in Umfangsrichtung zumindest über einen Teil des Umfanges variiert.

4. Verbindungseinrichtung (6) nach einem der vorstehenden Ansprüche, **dadurch gekennzeichnet, dass** die erste Kontaktfläche (26) zumindest in einem Bereich relativ zu der Drehachse geneigt ist.

5. Verbindungseinrichtung (6) nach einem der vorstehenden Ansprüche, **dadurch gekennzeichnet, dass** das erste Bauteil (2) und/oder das zweite Bauteil (4) relativ zu der Verbindungseinrichtung (6) schwenkbar ist, wenn es an dem jeweiligen Schwenkelement (8, 12) angeordnet ist.

6. Verbindungseinrichtung (6) nach Anspruch 5, **dadurch gekennzeichnet, dass** das erste Einstellelement (10) drehfest mit dem ersten Bauteil (2) verbindbar ist.

7. Verbindungseinrichtung (6) nach einem der vorstehenden Ansprüche, **dadurch gekennzeichnet, dass** an dem zweiten Schwenkelement (12) ein zweites Einstellelement (54) angeordnet ist, das die zweite Kontaktfläche (38) aufweist, wobei das zweite Einstellelement (54) vorzugsweise lösbar und/oder um eine zweite Drehachse drehbar angeordnet ist.

8. Gelenkeinrichtung (1) für eine orthopädietechnische Einrichtung, wobei die Gelenkeinrichtung (1)
- ein erstes Bauteil (2),
- ein zweites Bauteil (4) und
- eine Verbindungseinrichtung (6) nach einem der vorstehenden Ansprüche aufweist,
wobei das erste Bauteil (2) an dem ersten Schwenkelement (8) und das zweite Bauteil (4) an dem zweiten Schwenkelement (12) derart angeordnet sind, dass das erste Bauteil (2) relativ zu dem zweiten Bauteil (4) um eine Gelenkachse schwenkbar ist, wobei die Gelenkachse vorzugsweise einen rechten Winkel mit der Schwenkachse (14) einschließt.

9. Gelenkeinrichtung (1) nach Anspruch 8, **dadurch gekennzeichnet, dass** das erste Bauteil (2) und das zweite Bauteil (4) derart miteinander verbunden sind, dass durch ein Verschwenken eines der beiden Bauteile (2, 4) relativ zu der Verbindungseinrichtung (6) das jeweils andere der beiden Bauteile (4, 2) relativ zu der Verbindungseinrichtung (6) verschwenkt wird.

10. Gelenkeinrichtung (1) nach Anspruch 9, **dadurch gekennzeichnet, dass** die beiden Bauteile (2, 4) jeweils eine Mehrzahl von Zähnen (16) aufweisen, die ineinander eingreifen.

11. Gelenkeinrichtung (1) nach Anspruch 9 oder 10, **dadurch gekennzeichnet, dass** die beiden Bauteile (2, 4) durch wenigstens ein, bevorzugt wenigstens zwei Zugkraftübertragungselemente miteinander verbunden sind.

12. Gelenkeinrichtung (1) nach einem der Ansprüche 8 bis 11, **dadurch gekennzeichnet, dass** ein maximaler Verschwenkwinkel des ersten Bauteils (2) relativ zu dem zweiten Bauteil (4) in wenigstens einer Schwenkrichtung durch einen vorzugsweise einstellbaren Endanschlag (60) begrenzt ist.

## Claims

1. A connection device (6) for connecting a first component (2) to a second component (4) of an orthopedic device, wherein the connection device (6) comprises
- a first swivel element (8) for arranging on the first component (2) and
- a second swivel element (12) for arranging on the second component (4),
wherein
- a first adjustment element (10) is arranged on the first swivel element (8), said adjustment element having a first contact surface (26),
- the second swivel element (12) has a second contact surface (38),
wherein the first swivel element (8) is arranged on the second swivel element (12) such that it can be swivelled about a swivel axis (14) and the first adjustment element (10) is arranged and designed in such a way that the first contact surface (26) lies flat on the second contact surface (38) **characterized by** the fact that the first adjustment element (10) is arranged such that it can be rotated about a rotational axis, wherein a radial distance of the first contact surface (26) from the rotational axis varies.

2. The connection device (6) according to claim 1, **characterized by** the fact that the first adjustment element (10) is arranged on the first swivel element (8) such that it can be detached.

3. The connection device (6) according to one of the above claims, **characterized by** the fact that the radial distance of the first contact surface (26) from the rotational axis in the circumferential direction varies across at least one part of the circumference.

4. The connection device (6) according to one of the above claims, **characterized by** the fact that the first contact surface (26) is inclined relative to the rotational axis in at least one area.

5. The connection device (6) according to one of the above claims, **characterized by** the fact that the first component (2) and/or the second component (4) can be swivelled relative to the connection device (6) when it is arranged on the respective swivel element (8, 12).

6. The connection device (6) according to claim 5, **characterized by** the fact that the first adjustment element (10) can be connected to the first component (2) such that it is torque-proof.

7. The connection device (6) according to one of the above claims, **characterized by** the fact that a second adjustment element (54) is arranged on the second swivel element (12), said second adjustment element featuring the second contact surface (38), wherein the second adjustment element (54) is preferably arranged such that it can be detached and/or rotated about a second rotational axis.

8. A joint device (1) for an orthopedic device, wherein the joint device (1) comprises
- a first component (2),
- a second component (4) and
- a connection device (6) according to one of the above claims,
wherein the first component (2) is arranged on the first swivel element (8) and the second component (4) is arranged on the second swivel element (12) such that the first component (2) can be swivelled relative to the second component (4) about a joint axis, wherein the joint axis preferably forms a right angle with the swivel axis (14).

9. The joint device (1) according to claim 8, **characterized by** the fact that the first component (2) and the second component (4) are connected to one another in such a way that a swivelling of one of the two components (2, 4) relative to the connection device (6) effects a swivelling of the respective other of the two components (4, 2) relative to the connection device (6).

10. The joint device (1) according to claim 9, **characterized by** the fact that the two components (2, 4) each feature a multitude of teeth (16) that engage with each other.

11. The joint device (1) according to claim 9 or 10, **characterized by** the fact that the two components (2, 4) are connected to one another by way of at least one, preferably at least two, tensile force transmission elements.

12. The joint device (1) according to one of the claims 8 to 11, **characterized by** the fact that a maximum swivel angle of the first component (2) relative to the second component (4) is restricted in at least one swivel direction by a preferably adjustable end stop (60).

## Revendications

1. Dispositif de liaison (6) pour relier un premier composant (2) à un deuxième composant (4) d'un dispositif orthopédique, le dispositif de liaison (6) comprenant
- un premier élément pivotant (8) destiné à être disposé sur le premier composant (2), et
- un deuxième élément pivotant (12) destiné à être disposé sur le deuxième composant (4),
dans lequel
- un premier élément de réglage (10) est disposé sur le premier élément pivotant (8) et présente une première surface de contact (26),
- le deuxième élément pivotant (12) présente une deuxième surface de contact (38),
le premier élément pivotant (8) étant disposé sur le deuxième élément pivotant (12) de manière à pouvoir pivoter autour d'un axe de pivotement (14), et le premier élément de réglage (10) étant disposé et réalisé de telle sorte que la première surface de contact (26) s'applique contre la deuxième surface de contact (38),
**caractérisé en ce que**
le premier élément de réglage (10) est disposé de manière à pouvoir tourner autour d'un axe de rotation, et une distance radiale de la première surface de contact (26) par rapport à l'axe de rotation varie.

2. Dispositif de liaison (6) selon la revendication 1,
**caractérisé en ce que** le premier élément de réglage (10) est disposé de manière amovible sur le premier élément pivotant (8).

3. Dispositif de liaison (6) selon l'une des revendications précédentes, **caractérisé en ce que** la distance radiale de la première surface de contact (26) par rapport à l'axe de rotation varie dans la direction circonférentielle sur au moins une partie de la circonférence.

4. Dispositif de liaison (6) selon l'une des revendications précédentes, **caractérisé en ce que** la première surface de contact (26) est inclinée par rapport à l'axe de rotation au moins dans une zone.

5. Dispositif de liaison (6) selon l'une des revendications précédentes, **caractérisé en ce que** le premier composant (2) et/ou le deuxième composant (4) peut pivoter par rapport au dispositif de liaison (6) lorsqu'il est disposé sur l'élément pivotant respectif (8, 12).

6. Dispositif de liaison (6) selon la revendication 5,
**caractérisé en ce que** le premier élément de réglage (10) peut être relié solidairement en rotation au premier composant (2).

7. Dispositif de liaison (6) selon l'une des revendications précédentes, **caractérisé en ce qu'**un deuxième élément de réglage (54) présentant la deuxième surface de contact (38) est disposé sur le deuxième élément pivotant (12), le deuxième élément de réglage (54) étant disposé de préférence de manière amovible et/ou de manière à pouvoir tourner autour d'un deuxième axe de rotation.

8. Dispositif d'articulation (1) pour un dispositif orthopédique, le dispositif d'articulation (1) comprenant
- un premier composant (2),
- un deuxième composant (4) et
- un dispositif de liaison (6) selon l'une des revendications précédentes,
le premier composant (2) étant disposé sur le premier élément pivotant (8) et le deuxième composant (4) étant disposé sur le deuxième élément pivotant (12) de telle sorte que le premier composant (2) peut pivoter par rapport au deuxième composant (4) autour d'un axe d'articulation, l'axe d'articulation formant de préférence un angle droit avec l'axe de pivotement (14).

9. Dispositif d'articulation (1) selon la revendication 8,
**caractérisé en ce que** le premier composant (2) et le deuxième composant (4) sont reliés entre eux de telle sorte que, par un pivotement de l'un des deux composants (2, 4) par rapport au dispositif de liaison (6), l'autre des deux composants (4, 2) respectifs est pivoté par rapport au dispositif de liaison (6).

10. Dispositif d'articulation (1) selon la revendication 9,
**caractérisé en ce que** les deux composants (2, 4) présentent chacun une pluralité de dents (16) qui s'engrènent les unes dans les autres.

11. Dispositif d'articulation (1) selon la revendication 9 ou 10,
**caractérisé en ce que** les deux composants (2, 4) sont reliés entre eux par au moins un, de préférence au moins deux éléments de transmission de force de traction.

12. Dispositif d'articulation (1) selon l'une des revendications 8 à 11,
**caractérisé en ce qu'**un angle de pivotement maximal du premier composant (2) par rapport au deuxième composant (4) dans au moins une direction de pivotement est limité par une butée de fin de course (60), de préférence réglable.
